# EUROPÄISCHE PATENTSCHRIFT

(11) **EP 2 459 164 B1**
(45) Veröffentlichungstag und Bekanntmachung des Hinweises auf die Patenterteilung: **31.01.2018**
(21) Anmeldenummer: 10735018.3
(22) Anmeldetag: 28.07.2010
(51) Int. Cl.: A61K 8/73, A61K 8/86, A61Q 5/02, A61Q 19/10

(54) **KOSMETISCHES REINIGUNGSMITTEL**
COSMETIC CLEANING AGENT
NETTOYANT COSMÉTIQUE

(30) Priorität: 28.07.2009 DE 102009028052
(43) Veröffentlichungstag der Anmeldung: 06.06.2012
(73) Patentinhaber: Henkel AG & Co. KGaA, 40589 Düsseldorf (DE)
(72) Erfinder: CLAAS, Marcus, 40723 Hilden (DE); BANOWSKI, Bernhard, 40597 Düsseldorf (DE)
(86) Internationale Anmeldenummer: PCT/EP2010/060963
(87) Internationale Veröffentlichungsnummer: WO 2011/012650

(56) Entgegenhaltungen:
- EP-A2- 0 083 223
- EP-B1- 1 771 152
- WO-A1-98/00494
- WO-A2-98/04241
- US-A1- 2005 043 194
- AMERCHOL DOX CHEMICAL SOCIETY: "POLYOX Water-Soluble Resins", INTERNET CITATION, Mai 2002 (2002-05), XP002343601, Gefunden im Internet: URL:http://www.dow.com/PublishedLiterature /01b6/09002f13801b650c.pdf [gefunden am 2005-09-05]

## Beschreibung

Die vorliegende Erfindung liegt auf dem Gebiet der kosmetischen Mittel und betrifft eine Reinigungszusammensetzung auf der Basis einer speziellen Wirkstoffmischung.

Kosmetische Reinigungsmittel für die Haut und die Haare wie flüssige Seifen, Shampoos, Duschbäder, Schaumbäder sowie Dusch- und Waschgele müssen neben einem guten Reinigungsvermögen auch eine gute Verträglichkeit auf der Haut und den Schleimhäuten aufweisen, und auch bei häufiger Anwendung nicht zu starker Entfettung oder Hauttrockenheit führen.

Aus diesem Grund wird seit vielen Jahren versucht, möglichst viele Haar- und Hautkonditioniermittel in die Reinigungsmittel einzuarbeiten.

Es ist eine Vielzahl solcher 2in1-Produkte bekannt, doch die Lagerung und Stabilisierung der Produkte ist äußerst schwierig und stellt die Entwickler vor immer neue Herausforderungen. So lassen sich beispielsweise haut- und haarpflegende Inhaltsstoffe (Ölkomponenten oder spezielle Pflegepolymere) in kosmetische Reinigungsmittel einarbeiten, doch ihre stabile Dispergierung oder Emulgierung über einen langen Zeitraum ist oftmals problematisch.

Ein weiteres Problem kann darin bestehen, dass ionisch geladene Pflegekomponenten mit entgegengesetzt geladenen ionischen Komponenten in negativer Weise in Wechselwirkung treten, was wiederum eine Destabilisierung der Reinigungszusammensetzungen zur Folge haben kann. Ein weiterer Nachteil handelsüblicher 2in1-Reinigungszusammensetzungen besteht darin, dass sich die Anwesenheit einer Vielzahl von Pflegekomponenten in dem Reinigungsmittel negativ auf dessen Schaumeigenschaften auswirken kann, was von den Verbrauchern im Allgemeinen nicht akzeptiert wird.

In der EP 1771152A1 werden haarkonditionierende Mittel offenbart, die hochmolekulare Ethylenoxide und nichtionische Celluloseether enthalten, und den Haargriff nasser Haare verbessern. Die Schaumeigenschaften der haarkonditionmierenden Mittel werden nicht offenbart. Es besteht daher weiterhin der Bedarf nach kosmetischen 2in1-Haut- und Haarreinigungsmitteln, die stabil sind und über hervorragende Schaumeigenschaften verfügen.

Unter hervorragenden Schaumeigenschaften werden insbesondere eine hohe Schaummenge, eine rasche Bildung des Schaums sowie verbesserte sensorische und optische Eigenschaften des Schaums verstanden.

Vorgeschlagen werden daher kosmetische Reinigungsmittel, die aufgrund einer speziellen Wirkstoffkombination die Haut und das Haar gründlich und schonend reinigen, und hervorragende Schaumeigenschaften aufweisen.

Gegenstand der vorliegenden Erfindung ist ein kosmetisches Reinigungsmittel, enthaltend - bezogen auf sein Gewicht -
a) mindestens ein mildes anionisches und mindestens ein mildes amphoteres/zwitterionisches Tensid, wobei die Gesamtmenge an mildem(n) anionischem(n) und mildem(n) amphoterem(n)/zwitterionischem(n) Tensid(en) 3 bis 20 Gew.-% beträgt,
b) 0,005 bis 5 Gew.-% mindestens eines nichtionischen Celluloseethers und
c) 0,005 bis 5 Gew.-% mindestens eines nichtionischen Polymers der Formel (I) 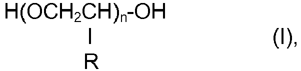
in der R für ein Wasserstoffatom oder eine Methylgruppe, und n für einen durchschnittlichen Wert von 10 bis 600 steht.

Erfindungsgemäß bevorzugte Reinigungsmittel enthalten mindestens ein mildes anionisches Tensid und mindestens ein mildes amphoteres/zwitterionisches Tensid in einem Verhältnis von anionischem(n) Tensid(en) zu dem(n) amphoteren/zwitterionischen Tensid(en) von 3 : 1 bis 1 : 2, bevorzugt 2,5 : 1 bis 1 : 1,5 und insbesondere 2 : 1 bis 1 : 1.

Als anionische Tenside eignen sich in erfindungsgemäßen Zubereitungen insbesondere
- lineare und verzweigte Fettsäuren mit 8 bis 30 C-Atomen (Seifen),
- Ethercarbonsäuren der Formel R-O-(CH₂-CH₂O)ₓ-CH₂-COOH, in der R eine lineare Alkylgruppe mit 8 bis 30 C-Atomen und x = 1 bis 16 ist,
- Acylsarcoside mit 8 bis 24 C-Atomen in der Acylgruppe,
- Acyltauride mit 8 bis 24 C-Atomen in der Acylgruppe,
- Acylisethionate mit 8 bis 24 C-Atomen in der Acylgruppe,
- Sulfobernsteinsäuremono- und -dialkylester mit 8 bis 24 C-Atomen in der Alkylgruppe und Sulfobernsteinsäuremono-alkylpolyoxyethylester mit 8 bis 24 C-Atomen in der Alkylgruppe und 1 bis 6 Oxyethylgruppen,
- lineare Alkansulfonate mit 8 bis 24 C-Atomen,
- lineare Alpha-Olefinsulfonate mit 8 bis 24 C-Atomen,
- Alpha-Sulfofettsäuremethylester von Fettsäuren mit 8 bis 30 C-Atomen,
- Alkylsulfate und Alkylpolyglykolethersulfate der Formel R-O(CH₂-CH₂O)ₓ-OSO₃H, in der R eine bevorzugt lineare Alkylgruppe mit 8 bis 30 C-Atomen und x = 0 oder 1 bis 12 ist,
- Gemische oberflächenaktiver Hydroxysulfonate,
- sulfatierte Hydroxyalkylpolyethylen- und/oder Hydroxyalkylenpropylenglykolether,
- Sulfonate ungesättigter Fettsäuren mit 8 bis 24 C-Atomen und 1 bis 6 Doppelbindungen,
- Ester der Weinsäure und Zitronensäure mit Alkoholen, die Anlagerungsprodukte von etwa 2-15 Molekülen Ethylenoxid und/oder Propylenoxid an Fettalkohole mit 8 bis 22 C-Atomen darstellen,
- Alkyl- und/oder Alkenyletherphosphate der Formel (TI), 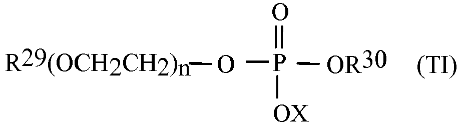 in der R²⁹ bevorzugt für einen aliphatischen Kohlenwasserstoffrest mit 8 bis 30 Kohlenstoffatomen, R³⁰ für Wasserstoff, einen Rest (CH₂CH₂O)ₙR²⁹ oder X, n für Zahlen von 1 bis 10 und X für Wasserstoff, ein Alkali- oder Erdalkalimetall oder NR³¹R³²R³³R³⁴, mit R³¹ bis R³⁴ unabhängig voneinander stehend für einen C₁ bis C₄ - Kohlenwasserstoffrest, steht,
- sulfatierte Fettsäurealkylenglykolester der Formel (TII) R³⁵CO(AlkO)ₙSO₃M (TII)
   in der R³⁵CO- für einen linearen oder verzweigten, aliphatischen, gesättigten und/oder ungesättigten Acylrest mit 6 bis 22 C-Atomen, Alk für CH₂CH₂, CHCH₃CH₂ und/oder CH₂CHCH₃, n für Zahlen von 0,5 bis 5 und M für ein Kation steht,
- Monoglyceridsulfate und Monoglyceridethersulfate der Formel (TIII), 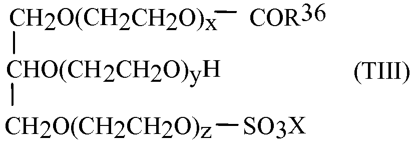
in der R³⁶CO für einen linearen oder verzweigten Acylrest mit 6 bis 22 Kohlenstoffatomen, x, y und z in Summe für 0 oder für Zahlen von 1 bis 30, vorzugsweise 2 bis 10, und X für ein Alkali- oder Erdalkalimetall steht. Typische Beispiele für im Sinne der Erfindung geeignete Monoglycerid(ether)sulfate sind die Umsetzungsprodukte von Laurinsäuremonoglycerid, Kokosfettsäuremonoglycerid, Palmitinsäuremonoglycerid, Stearinsäuremonoglycerid, Ölsäuremonoglycerid und Talgfettsäuremonoglycerid sowie deren Ethylenoxidaddukte mit Schwefeltrioxid oder Chlorsulfonsäure in Form ihrer Natriumsalze.
Vorzugsweise werden Monoglyceridsulfate der Formel (TIII) eingesetzt, in der R³⁶CO für einen linearen Acylrest mit 8 bis 18 Kohlenstoffatomen steht.

In den erfindungsgemäßen Reinigungszusammensetzungen werden bevorzugt Alkylpolyglykolethersulfate, Ethercarbonsäuren und/oder Sulfobernsteinsäuremono-alkylpolyoxyethylester mit 8 bis 18 C-Atomen in der Alkylgruppe und 1 bis 10, bevorzugt 1 bis 4 Oxethylgruppen im Molekül als milde anionische Tenside eingesetzt.

Das oder die anionische(n) Tensid(e) wird(werden) in den erfindungsgemäßen Zusammensetzungen - bezogen auf deren Gewicht - bevorzugt in Mengen von 1 bis 20 Gew.-% und insbesondere in Mengen von 2 bis 15 Gew.-% eingesetzt.

Alkylpolyglycolethersulfate mit 10 bis 18 C-Atomen in der Alkylgruppe und 1 bis 3 Glycolethergruppen im Molekül sind aufgrund ihrer milden Eigenschaften auf der Haut besonders bevorzugte anionische Tenside.

Als zwitterionische Tenside werden solche oberflächenaktiven Verbindungen bezeichnet, die im Molekül mindestens eine quartäre Ammoniumgruppe und mindestens eine -COO⁽⁻⁾ - oder -SO₃⁽⁻⁾ - Gruppe tragen. Besonders geeignete zwitterionische Tenside sind die sogenannten Betaine wie die N-Alkyl-N,N-dimethylammonium-glycinate, beispielsweise das Kokosalkyl-dimethylammoniumglycinat, N-Acyl-aminopropyl-N,N-dimethylammoniumglycinate, beispielsweise das Kokosacylaminopropyl-dimethylammoniumglycinat, und 2-Alkyl-3-carboxymethyl-3-hydroxyethyl-imidazoline mit jeweils 8 bis 18 C-Atomen in der Alkyl- oder Acylgruppe sowie das Kokosacylaminoethylhydroxyethylcarboxymethylglycinat. Besonders bevorzugte zwitterionische Tenside sind das unter der INCI-Bezeichnung Cocamidopropyl Betaine bekannte Fettsäureamid-Derivat sowie Alkylbetaine mit 10 bis 20 C-Atomen in der Alkylgruppe.

Unter ampholytischen Tensiden werden solche oberflächenaktiven Verbindungen verstanden, die außer einer C₈ - C₂₄ - Alkyl- oder -Acylgruppe im Molekül mindestens eine freie Aminogruppe und mindestens eine -COOH- oder -SO₃H-Gruppe enthalten und zur Ausbildung innerer Salze befähigt sind. Beispiele für geeignete ampholytische Tenside sind N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren mit jeweils etwa 8 bis 24 C-Atomen in der Alkylgruppe. Besonders bevorzugte ampholytische Tenside sind das N-Kokosalkylaminopropionat, das Kokosacylaminoethylaminopropionat und das C₁₂ - C₁₈ - Acylsarcosin.

Besonders bevorzugte amphotere/zwitterionische Tenside sind aufgrund ihrer milden Eigenschaften auf der Haut die C₈₋₁₈-Alkylbetaine, C₈₋₁₈-Alkylamido(C₁₋₄)-alkylbetaine und/oder C₈₋₁₈-Alkylamphomono- oder -diacetate.

Das oder die amphotere(n)/zwitterionische(n) Tensid(e) werden in den erfindungsgemäßen Zusammensetzungen - bezogen auf deren Gewicht - bevorzugt in Mengen von 0,5 bis 10 Gew.-% und insbesondere in Mengen von 1 bis 7,5 Gew.-% eingesetzt.

In einer besonders bevorzugten Ausführungsform der Erfindung enthalten die kosmetischen Reinigungsmittel eine Mischung aus Alkylethersulfaten und Alkylamidoalkylbetainen (wie bereits definiert). Diese Tenside wirken besonders mild auf der Haut und weisen eine vorteilhafte Schaumentwicklung und Schaumqualität auf.

Zur weiteren Unterstützung der schonenden Reinigung kann es bevorzugt sein, wenn die Reinigungsmittel weiterhin nichtionische Tenside enthalten. Diese beinhalten als hydrophile Gruppe z. B. eine Polyolgruppe, eine Polyalkylenglykolethergruppe oder eine Kombination aus Polyol- und Polyglykolethergruppe. Solche Verbindungen sind beispielsweise
- Anlagerungsprodukte von 2 bis 50 Mol Ethylenoxid und/oder 0 bis 5 Mol Propylenoxid an lineare und verzweigte Fettalkohole mit 8 bis 30 C-Atomen, an Fettsäuren mit 8 bis 30 C-Atomen und an Alkylphenole mit 8 bis 15 C-Atomen in der Alkylgruppe,
- mit einem Methyl- oder C₂ - C₆ - Alkylrest endgruppenverschlossene Anlagerungsprodukte von 2 bis 50 Mol Ethylenoxid und/oder 0 bis 5 Mol Propylenoxid an lineare und verzweigte Fettalkohole mit 8 bis 30 C-Atomen, an Fettsäuren mit 8 bis 30 C-Atomen und an Alkylphenole mit 8 bis 15 C-Atomen in der Alkylgruppe, wie beispielsweise die unter den Verkaufsbezeichnungen Dehydol® LS, Dehydol® LT (Cognis) erhältlichen Typen,
- Anlagerungsprodukte von 5 bis 60 Mol Ethylenoxid an Rizinusöl und gehärtetes Rizinusöl,
- Polyolfettsäureester, wie beispielsweise das Handelsprodukt Hydagen® HSP (Cognis) oder Sovermol - Typen (Cognis),
- alkoxilierte Triglyceride,
- alkoxilierte Fettsäurealkylester der Formel R³⁷CO-(OCH₂CHR³⁸)_{w}OR³⁹, (TIV), in der R³⁷CO für einen linearen oder verzweigten, gesättigten und/oder ungesättigten Acylrest mit 6 bis 22 Kohlenstoffatomen, R³⁸ für Wasserstoff oder Methyl, R³⁹ für lineare oder verzweigte Alkylreste mit 1 bis 4 Kohlenstoffatomen und w für Zahlen von 1 bis 20 steht,
- Aminoxide,
- Sorbitanfettsäureester und Anlagerungeprodukte von Ethylenoxid an Sorbitanfettsäureester wie beispielsweise die Polysorbate,
- Zuckerfettsäureester und Anlagerungsprodukte von Ethylenoxid an Zuckerfettsäureester,
- Fettsäure-N-alkylglucamide.

Eine weitere Gruppe geeigneter, nichtionischer Tenside sind die Alkylpolyglucoside. Sie entsprechen der Formel (I)

R¹O-[G]ₚ (I)

in der R¹ für einen Alkyl- und/oder Alkenylrest mit 4 bis 22 Kohlenstoffatomen, G für einen Zuckerrest mit 5 oder 6 Kohlenstoffatomen und p für Zahlen von 1 bis 10 steht. Die Indexzahl p in der allgemeinen Formel (I) gibt den Oligomerisierungsgrad (DP), d.h. die Verteilung von Mono- und Oligoglycosiden an und steht für eine Zahl zwischen 1 und 10. Während p in einer gegebenen Verbindung stets ganzzahlig sein muss und hier vor allem die Werte p = 1 bis 6 annehmen kann, ist der Wert p für ein bestimmtes Alkyloligoglycosid eine analytisch ermittelte rechnerische Größe, die meistens eine gebrochene Zahl darstellt. Vorzugsweise werden Alkyl- und/oder Alkenyloligoglycoside mit einem mittleren Oligomerisierungsgrad p von 1,1 bis 3,0 eingesetzt. Aus anwendungstechnischer Sicht sind solche Alkyl- und/oder Alkenyloligoglycoside bevorzugt, deren Oligomerisierungsgrad kleiner als 1,7 ist und insbesondere zwischen 1,2 und 1,7 liegt. Der Alkyl- bzw. Alkenylrest R¹ kann sich von primären Alkoholen mit 4 bis 11, vorzugsweise 8 bis 10 Kohlenstoffatomen ableiten. Erfindungsgemäß ganz besonders bevorzugt sind Alkyloligoglucoside auf der Basis von gehärtetem C_{12/14}-Kokosalkohol mit einem DP von 1-3, wie sie beispielsweise unter der INCI-Bezeichnung "Coco-Glucoside" im Handel erhältlich sind.

Die nichtionischen Tenside werden in den erfindungsgemäßen Zusammensetzungen - bezogen auf deren Gewicht - bevorzugt in Mengen von 0,05 bis 15 Gew.-%, mehr bevorzugt von 0,1 bis 10 Gew.-% und insbesondere in Mengen von 0,5 bis 5 Gew.-% eingesetzt.

Als zweite zwingende Komponente b) enthalten die erfindungsgemäßen Reinigungsmittel einen nichtionischen Celluloseether, vorzugsweise aus der Gruppe der Ether und Mischether, der bevorzugt wasserlöslich ist.

Unter der Bezeichnung "wasserlöslich" sind solche Celluloseether zu verstehen, deren 1%-ige Lösung in Wasser bei 25°C für das menschliche Auge klar oder transluzent ist.

Bevorzugte Celluloseether sind Hydroxyethylcellulose, Hydroxypropylcellulose und/oder Hydroxypropyl Methylcellulose sowie Gemische dieser Stoffe. Sie sind im Handel mit verschiedenen Viskositäten erhältlich.

Die geeigneten Celluloseether begünstigen die guten Schaumeigenschaften der erfindungsgemäßen Reinigungsmittel und verleihen Ihnen vorteilhafte rheologische-Eigenschaften. Es wurde gefunden, dass die erfindungsgemäßen Reinigungsmittel besonders stabil und schaumstark sind, wenn sie eine höhere Viskosität aufweisen.

Bevorzugte Reinigungsmittel weisen daher eine Viskosität im Bereich von 5000 bis 15000 mPas, bevorzugt von 6000 bis 12000 mPas und insbesondere von 7500 bis 10500 mPas auf (jeweils gemessen mit einem Haake Viskosimeter Viscotester VT550; Temperatur: 20°C, Messeinrichtung: Zylinder MK-2; Schwergeschwindigkeit 8/sek.).

Erfindungsgemäß bevorzugte Reinigungsmittel enthalten Hydroxypropyl Methylcellulose, wie sie beispielsweise von der Firma Dow Chemicals unter der Bezeichnung "Methocel®" oder von der Firma Hercules unter der Bezeichnung "Benecel®" mit verschiedenen Viskositätsgraden im Handel erhältlich ist. Eine für die erfindungsgemäßen Reinigungsmittel geeignete Viskosität der Celluloseether liegt im Bereich von 100 bis 100,000 mPas, bevorzugt im Bereich von 2,000 bis 10,000 mPas (bezogen auf eine 2%ige Lösung des jeweiligen Celluloseethers in Wasser bei 20 °C; gemessen mit einem Ubbelohde tube Viskosimeter).

Erfindungsgemäß besonders bevorzugte Reinigungsmittel enthalten Hydroxypropyl Methylcellulose, wie sie beispielsweise im Handel unter der Bezeichnung "Methocel®" (E-, F-, J-, K- und 40-Serien) von der Firma Dow Chemical oder "Benecel®" von der Firma Hercules erhältlich ist. Insbesondere bevorzugt sind die Handelsprodukte Methocel® 40-202, Methocel® E4MP, Methocel® 40-100, Methocel® 40-101 und Benecel® MP 330C.

Die nichtionischen Celluloseether b) werden in den erfindungsgemäßen Reinigungsmitteln - bezogen auf deren Gewicht - bevorzugt in Mengen von 0,01 bis 4 Gew.-%, mehr bevorzugt von 0,05 bis 3 Gew.-% und insbesondere von 0,1 bis 2 Gew.-% eingesetzt.

Geeignete nichtionische Polymere c) der erfindungsgemäßen Reinigungsmittel sind bevorzugt ebenfalls wasserlöslich, wobei unter der Bezeichnung "wasserlöslich" solche nichtionischen Polymere c) zu verstehen sind, deren 1%-ige Lösung in Wasser bei 25°C für das menschliche Auge klar oder transluzent ist.

Geeignete nichtionische Polymere c) entsprechen der Formel (I), 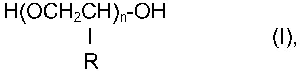 in der R für ein Wasserstoffatom oder eine Methylgruppe, und n im Mittel für eine ganze Zahl zwischen 10 und 1200 steht. Besonders bevorzugte Polymere c) weisen einen Wert n von 10 600 und insbesondere von 10 bis 400 auf. Insbesondere bevorzugt sind Polyethylenglycole mit Molgewichten im Bereich von 300 bis 25000 Dalton, bevorzugt von 500 bis 20000 Dalton und insbesondere von 1000 bis 15000 Dalton. Es wurde gefunden, dass diese speziellen Polymere c) die vorteilhafte Rheologie der erfindungsgemäßen Reinigungsmittel sowie deren Schaumeigenschaften, insbesondere die Feinporigkeit und Cremigkeit des Schaums, unterstützen.

Weiterhin wurde gefunden, dass sich die zuvor beschriebenen niedermolekularen Polyethylenglycole in den erfindungsgemäßen Reinigungsmitteln vorteilhaft auf die sensorischen Eigenschaften des Schaums auswirken, denn der Schaum hinterlässt auf der Haut kein glitschiges Gefühl.

Die nichtionischen Polymere c) werden in den erfindungsgemäßen Reinigungszusammensetzungen - bezogen auf deren Gewicht - bevorzugt in Mengen von 0,01 bis 4 Gew.-%, mehr bevorzugt von 0,05 bis 3 Gew.-% und insbesondere von 0,1 bis 2 Gew.-% eingesetzt.

In einer besonders bevorzugten Ausführungsform der Erfindung enthalten die erfindungemäßen Reinigungsmittel zur Unterstützung der Schaummenge und -dichte sowie zur weiteren Verbesserung der Schaumstabilität - bezogen auf ihr Gewicht - zusätzlich 0,01 bis 5 Gew.-%, bevorzugt 0,05 bis 4 Gew.-%, mehr bevorzugt 0,1 bis 3 Gew.-% und insbesondere 0,2 bis 2 Gew.-% mindestens eines Alkanolamids d) der Formel (II): 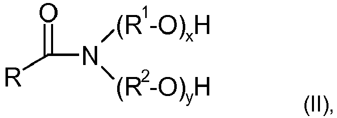 in der R eine C₈-C₂₄ gesättigte oder ungesättigte, lineare oder vernetzte aliphatische Gruppe, R₁ und R₂ gleich oder verschieden sind und eine C₂-C₄ lineare oder verzweigte aliphatische Gruppe bilden, x einen Wert von 0 bis 10 und y einen Wert von 1 bis 10 aufweist, wobei die Summe aus x+y kleiner oder gleich 10 ist.

Spezifische Beispiele geeigneter Alkanolamide sind beispielsweise die unter den INCI-Bezeichnungen "Cocamide MEA", "Cocamide DEA" und "Cocamide MIPA" bekannten Verbindungen.

In einer weiteren bevorzugten Ausführungsform der Erfindung enthalten die erfindungemäßen Reinigungsmittel zur Unterstützung der Rückfettung und Pflege der Haut zusätzlich Monoester und/oder Gemische aus Monoestern und Diestern des Glycerins mit verzweigten, oder geradkettigen, gesättigten oder ungesättigten Fettsäuren einer C-Kettenlänge von 8 bis 24, bevorzugt von 10 bis 18 und insbesondere von 12 bis 16, die einen Ethoxylierungsgrad von 1 bis 20, bevorzugt von 2 bis 17, besonders bevorzugt von 4 bis 13 und insbesondere von 6 bis 10 aufweisen. Erfindungsgemäß bevorzugt sind die ethoxylierten Glyceryloleate und Glycerylcocoate und insbesondere bevorzugt ist PEG-7 Glyceryl Cocoate, wie es beispielsweise im Handel unter der Bezeichnung Tegosoft® GC oder Cetiol® HE erhältlich ist.

Der pH-Wert der erfindungsgemäßen Reinigungszusammensetzungen liegt idealerweise in einem schonenden Bereich für die Haut von etwa 4 bis 6, insbesondere in einem Bereich von 4,5 bis 5,5. Neben den vorgenannten Bestandteilen können die Reinigungszusammensetzungen eine Reihe weiterer fakultativer Bestandteile enthalten. Bevorzugt werden den Reinigungszusammensetzungen weitere Wirkstoffe zugesetzt, die kosmetische Pflegeeigenschaften aufweisen, um die Konditionierung der Haut und/oder der Haare während des Reinigungsvorgangs zu unterstützen. Als solche sind insbesondere kosmetisch geeignete Ölkomponenten, Pflanzenextrakte und/oder Feuchthaltemittel als weitere bevorzugte fakultative Komponenten zu nennen.

Geeignete Ölkomponenten können ausgewählt sein aus mineralischen, natürlichen oder synthetischen Ölkomponenten wie Petrolatum, Paraffinen, Silikonen, Fettalkoholen, Fettsäuren, Fettsäureestern sowie natürlichen Ölen pflanzlichen und tierischen Ursprungs. Sie werden in den Reinigungszusammensetzungen - bezogen auf ihr Gesamtgewicht - bevorzugt in einer Menge von 0,01 bis 10 Gew.-%, besonders bevorzugt von 0,05 bis 5 Gew.-% und insbesondere von 0,2 bis 3 Gew.-% eingesetzt (wobei sich die Mengenangabe auf den Gesamtgehalt aller Ölkomponenten in den erfindungsgemäßen Reinigungsmittels bezieht).

Unter dem Begriff Silikonöle versteht der Fachmann mehrere Strukturen siliciumorganischer Verbindungen, die bevorzugt ausgewählt sind aus mindestens einem Vertreter siliciumorganischer Verbindungen, die gebildet wird aus:
(i) Polyalkylsiloxanen, Polyarylsiloxanen, Polyalkylarylsiloxanen, die flüchtig oder nicht flüchtig, geradkettig, verzweigt oder cyclisch, vernetzt oder nicht vernetzt sind;
(ii) Polysiloxanen, die in ihrer allgemeinen Struktur eine oder mehrere organofunktionelle Gruppen enthalten, die ausgewählt sind unter:
   a) substituierten oder unsubstituierten aminierten Gruppen;
   b) (per)fluorierten Gruppen;
   c) Thiolgruppen;
   d) Carboxylatgruppen;
   e) hydroxylierten Gruppen;
   f) alkoxylierten Gruppen;
   g) Acyloxyalkylgruppen;
   h) amphoteren Gruppen;
   i) Bisulfitgruppen;
   j) Hydroxyacylaminogruppen;
   k) Carboxygruppen;
   l) Sulfonsäuregruppen; und
   m) Sulfat- oder Thiosulfatgruppen;
(iii) linearen Polysiloxan(A)- Polyoxyalkylen(B)- Blockcopoylmeren vom Typ (A-B)ₙ mit n > 3;
(iv) gepfropften Silikonpolymeren mit nicht silikonhaltigem, organischen Grundgerüst, die aus einer organischen Hauptkette bestehen, welche aus organischen Monomeren gebildet wird, die kein Silikon enthalten, auf die in der Kette sowie gegebenenfalls an mindestens einem Kettenende mindestens ein Polysiloxanmakromer gepfropft wurde;
(v) gepfropften Silikonpolymeren mit Polysiloxan-Grundgerüst, auf das nicht silikonhaltige, organische Monomere gepfropft wurden, die eine Polysiloxan-Hauptkette aufweisen, auf die in der Kette sowie gegebenenfalls an mindestens einem ihrer Enden mindestens ein organisches Makromer gepfropft wurde, das kein Silikon enthält, wie beispielsweise das unter der INCI-Bezeichnung Bis-PEG/PPG-20/20 Dimethicone vertriebene Handelsprodukt Abil B 8832 der Firma Degussa;
(vi) oder deren Gemischen.

Bevorzugte haarkonditionierende Silikone werden ausgewählt aus Dimethiconen, Amodimethiconen oder Dimethiconolen.

Als Fettsäuren können eingesetzt werden lineare und/oder verzweigte, gesättigte und/oder ungesättigte Fettsäuren mit 6 - 30 Kohlenstoffatomen. Bevorzugt sind Fettsäuren mit 10 - 22 Kohlenstoffatomen. Hierunter wären beispielsweise zu nennen die Isostearinsäuren, wie die Handelsprodukte Emersol® 871 und Emersol® 875, und Isopalmitinsäuren wie das Handelsprodukt Edenor® IP 95, sowie alle weiteren unter den Handelsbezeichnungen Edenor® (Cognis) vertriebenen Fettsäuren. Weitere typische Beispiele für solche Fettsäuren sind Capronsäure, Caprylsäure, 2-Ethylhexansäure, Caprinsäure, Laurinsäure, Isotridecansäure, Myristinsäure, Palmitinsäure, Palmitoleinsäure, Stearinsäure, Isostearinsäure, Ölsäure, Elaidinsäure, Petroselinsäure, Linolsäure, Linolensäure, Elaeostearinsäure, Arachinsäure, Gadoleinsäure, Behensäure und Erucasäure sowie deren technische Mischungen.

Besonders bevorzugt sind üblicherweise die Fettsäureschnitte, welche aus Cocosöl oder Palmöl erhältlich sind; insbesondere bevorzugt ist in der Regel der Einsatz von Stearinsäure.

Als Fettalkohole können eingesetzt werden gesättigte, ein- oder mehrfach ungesättigte, verzweigte oder unverzweigte Fettalkohole mit C₆-C₃₀-, bevorzugt C₁₀-C₂₂- und ganz besonders bevorzugt C₁₂-C₂₂- Kohlenstoffatomen. Einsetzbar im Sinne der Erfindung sind beispielsweise Decanol, Octanol, Octenol, Dodecenol, Decenol, Octadienol, Dodecadienol, Decadienol, Oleylalkohol, Erucaalkohol, Ricinolalkohol, Stearylalkohol, Isostearylalkohol, Cetylalkohol, Laurylalkohol, Myristylalkohol, Arachidylalkohol, Caprylalkohol, Caprinalkohol, Linoleylalkohol, Linolenylalkohol und Behenylalkohol, sowie deren Guerbetalkohole, wobei diese Aufzählung beispielhaften und nicht limitierenden Charakter haben soll. Die Fettalkohole stammen jedoch von bevorzugt natürlichen Fettsäuren ab, wobei üblicherweise von einer Gewinnung aus den Estern der Fettsäuren durch Reduktion ausgegangen werden kann. Einsetzbar sind ebenfalls solche Fettalkoholschnitte, die durch Reduktion natürlich vorkommender Triglyceride wie Rindertalg, Palmöl, Erdnußöl, Rüböl, Baumwollsaatöl, Sojaöl, Sonnenblumenöl und Leinöl oder aus deren Umesterungsprodukten mit entsprechenden Alkoholen entstehenden Fettsäureestern erzeugt werden, und somit ein Gemisch von unterschiedlichen Fettalkoholen darstellen. Solche Substanzen sind beispielsweise unter den Bezeichnungen Stenol®, z.B. Stenol® 1618 oder Lanette®, z.B. Lanette® O oder Lorol®, z.B. Lorol® C8, Lorol® C14, Lorol® C18, Lorol® C8-18, HD-Ocenol®, Crodacol®, z.B. Crodacol® CS, Novol®, Eutanol® G, Guerbitol® 16, Guerbitol® 18, Guerbitol® 20, Isofol® 12, Isofol® 16, Isofol® 24, Isofol® 36, Isocarb® 12, Isocarb® 16 oder Isocarb® 24 käuflich zu erwerben. Selbstverständlich können auch Wollwachsalkohole, wie sie beispielsweise unter den Bezeichnungen Corona®, White Swan®, Coronet® oder Fluilan® käuflich zu erwerben sind, eingesetzt werden.

Als natürliche oder synthetische Wachse können feste Paraffine oder Isoparaffine, Carnaubawachse, Bienenwachse, Candelillawachse, Ozokerite, Ceresin, Walrat, Sonnenblumenwachs, Fruchtwachse wie beispielsweise Apfelwachs oder Citruswachs, Microwachse aus PE- oder PP eingesetzt werden. Derartige Wachse sind beispielsweise erhältlich über die Fa. Kahl & Co., Trittau.

Zu den natürlichen und synthetischen kosmetischen Ölkörpern sind beispielsweise zu zählen:
- flüssige Paraffinöle, Isoparaffinöle und synthetische Kohlenwasserstoffe sowie Di-n-alkylether mit insgesamt zwischen 12 bis 36 C-Atomen, insbesondere 12 bis 24 C-Atomen, wie beispielsweise Di-n-octylether, Di-n-decylether, Di-n-nonylether, Di-n-undecylether, Di-n-dodecylether, n-Hexyl-n-octylether, n-Octyl-n-decylether, n-Decyl-n-undecylether, n-Undecyl-n-dodecylether und n-Hexyl-n-Undecylether sowie Di-tert-butylether, Di-iso-pentylether, Di-3-ethyldecylether, tert.-Butyl-n-octylether, iso-Pentyl-n-octylether und 2-Methyl-pentyl-n-octylether. Die als Handelsprodukte erhältlichen Verbindungen 1,3-Di-(2-ethyl-hexyl)-cyclohexan (Cetiol® S) und Di-n-octylether (Cetiol® OE) können bevorzugt sein.
- Esteröle. Unter Esterölen sind zu verstehen die Ester von C₆-C₃₀ - Fettsäuren mit C₂-C₃₀ - Fettalkoholen. Bevorzugt sind die Monoester der Fettsäuren mit Alkoholen mit 2 bis 24 C-Atomen. Beispiele für eingesetzte Fettsäurenanteile in den Estern sind Capronsäure, Caprylsäure, 2-Ethylhexansäure, Caprinsäure, Laurinsäure, Isotridecansäure, Myristinsäure, Palmitinsäure, Palmitoleinsäure, Stearinsäure, Isostearinsäure, Ölsäure, Elaidinsäure, Petroselinsäure, Linolsäure, Linolensäure, Elaeostearinsäure, Arachinsäure, Gadoleinsäure, Behensäure und Erucasäure sowie deren technische Mischungen.
   Beispiele für die Fettalkoholanteile in den Esterölen sind Isopropylalkohol, Capronalkohol, Caprylalkohol, 2-Ethylhexylalkohol, Caprinalkohol, Laurylalkohol, Isotridecylalkohol, My ristylalkohol, Cetylalkohol, Palmoleylalkohol, Stearylalkohol, Isostearylalkohol, Oleylalkohol, Elaidylalkohol, Petroselinylalkohol, Linolylalkohol, Linolenylalkohol, Elaeostearylalkohol, Arachylalkohol, Gadoleylalkohol, Behenylalkohol, Erucylalkohol und Brassidylalkohol sowie he Mischungen.
   Besonders bevorzugt sind Isopropylmyristat (Rilanit® IPM), Isononansäure- C₁₆-₁₈-alkylester (Cetiol® SN), 2-Ethylhexylpalmitat (Cegesoft® 24), Stearinsäure-2-ethylhexylester (Cetiol® 868), Cetyloleat, Glycerintricaprylat, Kokosfettalkohol-caprinat/-caprylat (Cetiol® LC), n-Butylstearat, Oleylerucat (Cetiol® J 600), Isopropylpalmitat (Rilanit® IPP), Oleyl Oleate (Cetiol®), Laurinsäurehexylester (Cetiol® A), Di-n-butyladipat (Cetiol® B), Myristylmyristat (Cetiol® MM), Cetearyl Isononanoate (Cetiol® SN), Ölsäuredecylester (Cetiol® V).
- Dicarbonsäureester wie Di-n-butyladipat, Di-(2-ethylhexyl)-adipat, Di-(2-ethylhexyl)-succinat und Di-isotridecylacelaat sowie Diolester wie Ethylenglykol-dioleat, Ethylenglykol-di-isotridecanoat, Propylenglykol-di(2-ethylhexanoat), Propylenglykol-di-isostearat, Propylenglykol-di-pelargonat, Butandiol-di-isostearat, Neopentylglykoldicaprylat,
- symmetrische, unsymmetrische oder cyclische Ester der Kohlensäure mit Fettalkoholen, Glycerincarbonat oder Dicaprylylcarbonat (Cetiol® CC),
- Trifettsäureester von gesättigten und/oder ungesättigten linearen und/oder verzweigten Fettsäuren mit Glycerin,
- Gegebenenfalls ethoxylierte Fettsäurepartialglyceride, das sind gegebenenfalls ethoxylierte Monoglyceride, Diglyceride und deren technische Gemische. Bei der Verwendung technischer Produkte können herstellungsbedingt noch geringe Mengen Triglyceride enthalten sein. Die Partialglyceride folgen vorzugsweise der Formel, 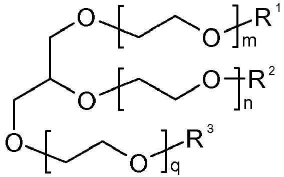
in der R¹, R² und R³ unabhängig voneinander für Wasserstoff oder für einen linearen oder verzweigten, gesättigten und/oder ungesättigten Acylrest mit 6 bis 22, vorzugsweise 12 bis 18, Kohlenstoffatomen stehen mit der Maßgabe, dass mindestens eine dieser Gruppen für einen Acylrest und mindestens eine dieser Gruppen für Wasserstoff steht. Die Summe (m+n+q) steht für 0 oder Zahlen von 1 bis 100, vorzugsweise für 0 oder 5 bis 25. Bevorzugt steht R1 für einen Acylrest und R2 und R3 für Wasserstoff und die Summe (m+n+q) ist 0. Typische Beispiele sind Mono- und/oder Diglyceride auf Basis von Capronsäure, Caprylsäure, 2-Ethylhexansäure, Caprinsäure, Laurinsäure, Isotridecansäure, Myristinsäure, Palmitinsäure, Palmoleinsäure, Stearinsäure, Isostearinsäure, Ölsäure, Elaidinsäure, Petroselinsäure, Linolsäure, Linolensäure, Elaeostearinsäure, Arachinsäure, Gadoleinsäure, Behensäure und Erucasäure sowie deren technische Mischungen. Vorzugsweise werden Ölsäuremonoglyceride eingesetzt.

In einer besonders bevorzugten Ausführungsform der Erfindung können die erfindungsgemäßen Reinigungsmittel als Ölkomponente ein pflanzliches Öl enthalten.

Als natürliche Öle kommen beispielsweise Amaranthsamenöl, Aprikosenkernöl, Arganöl, Avocadoöl, Babassuöl, Baumwollsaatöl, Borretschsamenöl, Camelinaöl, Distelöl, Erdnußöl, Granatapfelkernöl, Grapefruitsamenöl, Hanföl, Hagebuttenkernöl, Haselnussöl, Holundersamenöl, Johannisbeersamenöl, Jojobaöl, Kakaobutter, Leinöl, Macadamianussöl, Maiskeimöl, Mandelöl, Marulaöl, Nachtkerzenöl, Olivenöl, Palmöl, Pfirsichkernöl, Rapsöl, Reisöl, Sanddornfruchtfleischöl, Sanddornkernöl, Sesamöl, Sheabutter, Sojaöl, Sonnenblumenöl, Traubenkernöl, Walnußöl oder Wildrosenöl in Frage.

Besonders bevorzugt sind Avocadoöl, Aprikosenkernöl, Hagebuttenkernöl, Jojobaöl, Kakaobutter, Mandelöl, Olivenöl, Pfirsichkernöl, Sheabutter, Sonnenblumenöl und Traubenkernöl.

Unter geeigneten Pflanzenextrakten sind Extrakte zu verstehen, die aus allen Teilen einer Pflanze hergestellt werden können.

Üblicherweise werden diese Extrakte durch Extraktion der gesamten Pflanze hergestellt. Es kann aber in einzelnen Fällen auch bevorzugt sein, die Extrakte ausschließlich aus Blüten und/oder Blättern der Pflanze herzustellen.

Bevorzugt für den Einsatz in den erfindungsgemäßen Reinigungsmitteln sind vor allem die Extrakte aus Grünem Tee, Weißem Tee, Eichenrinde, Brennessel, Hamamelis, Hopfen, Kamille, Klettenwurzel, Schachtelhalm, Weißdorn, Lindenblüten, Litschi, Mandel, Aloe Vera, Fichtennadel, Roßkastanie, Sandelholz, Wacholder, Kokosnuß, Mango, Aprikose, Limone, Weizen, Kiwi, Melone, Orange, Grapefruit, Salbei, Rosmarin, Birke, Malve, Wiesenschaumkraut, Quendel, Schafgarbe, Thymian, Melisse, Hauhechel, Huflattich, Eibisch, Ginseng, Ingwerwurzel, Echinacea purpurea, Olea europea, Foeniculum vulgaris und Apim graveolens.

Als Extraktionsmittel zur Herstellung der genannten Pflanzenextrakte können Wasser, Alkohole sowie deren Mischungen verwendet werden. Unter Alkoholen sind dabei niedere Alkohole wie Ethanol und Isopropanol, insbesondere aber mehrwertige Alkohole wie Ethylenglykol und Propylenglykol, sowohl als alleiniges Extraktionsmittel als auch in Mischung mit Wasser, bevorzugt. Pflanzenextrakte auf Basis von Wasser/Propylenglykol im Verhältnis 1:10 bis 10:1 haben sich als besonders geeignet erwiesen.

Die Pflanzenextrakte können sowohl in reiner als auch in verdünnter Form eingesetzt werden. Sofern sie in verdünnter Form eingesetzt werden, enthalten sie üblicherweise ca. 2 - 80 Gew.-% Aktivsubstanz und als Lösungsmittel das bei ihrer Gewinnung eingesetzte Extraktionsmittel oder Extraktionsmittelgemisch.

Zusätzlich kann es sich als vorteilhaft erweisen, wenn die erfindungsgemäßen Zusammensetzungen Feuchthaltemittel bzw. Penetrationshilfsstoffe und/ oder Quellmittel (M) enthalten. Diese Hilfsstoffe sorgen für eine bessere Penetration von Wirkstoffen in die keratinische Faser oder helfen die keratinische Faser aufzuquellen. Zu den geeigneten Feuchthaltemitteln bzw. Penetrationshilfsstoffen und/ oder Quellmitteln (M) zählen beispielsweise Harnstoff und Harnstoffderivate, Guanidin und dessen Derivate, Arginin und dessen Derivate, Wasserglas, Imidazol und Dessen Derivate, Histidin und dessen Derivate, Benzylalkohol, Glycerin, Glykol und Glykolether, Propylenglykol und Propylenglykolether, beispielsweise Propylenglykolmonoethylether, Carbonate, Hydrogencarbonate, Diole und Triole, und insbesondere 1,2-Diole und 1,3-Diole wie beispielsweise 1,2-Propandiol, 1,2-Pentandiol, 1,2-Hexandiol, 1,2-Dodecandiol, 1,3-Propandiol, 1,6-Hexandiol, 1,5-Pentandiol, 1,4-Butandiol.

Besonders geeignet ist Glycerin.

Die Feuchthaltemittel bzw. Penetrationshilfsstoffe und/oder Quellmittel (M) werden in den erfindungsgemäßen Reinigungszusammensetzungen - bezogen auf die gesamte Zusammensetzung - bevorzugt in Mengen von 0,05 bis 5 Gew.-% und insbesondere in Mengen von 0,1 bis 3 Gew.-% eingesetzt.

Als weitere fakultative Komponenten können kationische Polymere in den erfindungsgemäßen Reinigungszusammensetzungen eingesetzt werden.

Geeignete kationische Polymere sind Polymere, welche in der Haupt- und/oder Seitenkette Gruppen aufweisen, welche "temporär" oder "permanent" kationisch sein können. Als "permanent kationisch" werden solche Polymere bezeichnet, die unabhängig vom pH-Wert des Mittels eine kationische Gruppe aufweisen. Dies sind in der Regel Polymere, die ein quartäres Stickstoffatom, beispielsweise in Form einer Ammoniumgruppe, enthalten.

Bevorzugte kationische Gruppen enthalten quartäre Ammoniumgruppen. Insbesondere solche Polymere, bei denen die quartäre Ammoniumgruppe über eine C₁₋₄-Kohtenwasserstoffgruppe an eine aus Acrylsäure, Methacrylsäure oder deren Derivaten aufgebaute Polymerhauptkette gebunden ist, haben sich als besonders geeignet erwiesen.

Homopolymere, enthaltend eine Gruppierung der allgemeinen Formel (VI), 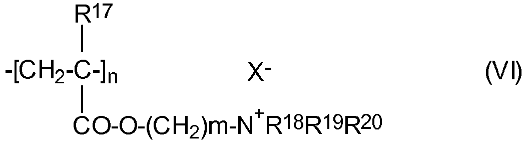 in der R¹⁷ = -H oder -CH₃ ist, R¹⁸, R¹⁹ und R²⁰ unabhängig voneinander ausgewählt sind aus C₁₋₄-Alkyl-, -Alkenyl- oder -Hydroxyalkylgruppen, m = 1, 2, 3 oder 4, n eine natürliche Zahl und X⁻ein physiologisch verträgliches organisches oder anorganisches Anion ist, sowie Copolymere, bestehend im wesentlichen aus den in Formel (VI) aufgeführten Monomereinheiten sowie nichtionogenen Monomereinheiten, sind besonders bevorzugte kationische Polymere.

Im Rahmen dieser Polymere sind diejenigen bevorzugt, für die mindestens eine der folgenden Bedingungen gilt:
- R¹⁷ steht für eine Methylgruppe
- R¹⁸, R¹⁹ und R²⁰ stehen für Methylgruppen
- m hat den Wert 2.

Als physiologisch verträgliches Gegenionen X⁻ kommen beispielsweise Halogenidionen, Sulfationen, Phosphationen, Methosulfationen sowie organische Ionen wie Lactat-, Citrat-, Tartrat- und Acetationen in Betracht. Bevorzugt sind Halogenidionen, insbesondere Chlorid.

Geeignete Homo- oder Copolymere, die sich von der Formel (VI) ableiten, sind beispielsweise die unter den Handelsbezeichnungen Salcare® SC 95, Salcare® SC 96 und Salcare® SC 92 im Handel erhältlich.

Weitere bevorzugte kationische Polymere sind beispielsweise
- quaternisierte Cellulose-Derivate, wie sie unter den Bezeichnungen Celquat® und Polymer JR® im Handel erhältlich sind. Die Verbindungen Celquat® H 100, Celquat® L 200 und Polymer JR®400 sind bevorzugte quaternierte Cellulose-Derivate,
- hydrophob modifizierte Cellulosederivate, beispielsweise die unter dem Handelsnamen SoftCat® vertriebenen kationischen Polymere,
- kationische Alkylpolyglycoside,
- kationiserter Honig, beispielsweise das Handelsprodukt Honeyquat® 50,
- kationische Guar-Derivate, wie insbesondere die unter den Handelsnamen Cos-media®Guar und Jaguar® vertriebenen Produkte,
- Polysiloxane mit quaternären Gruppen, wie beispielsweise die im Handel erhältlichen erhältlichen Produkte Q2-7224 (Hersteller: Dow Corning; ein stabilisiertes Trimethylsilylamodimethicon), Dow Corning® 929 Emulsion (enthaltend ein hydroxyl-aminomodifiziertes Silicon, das auch als Amodimethicone bezeichnet wird), SM-2059 (Hersteller: General Electric), SLM-55067 (Hersteller: Wacker) sowie Abil®-Quat 3270 und 3272 (Hersteller: Th. Goldschmidt; diquaternäre Polydimethylsiloxane, Quaternium-80),
- polymere Dimethyldiallylammoniumsalze und deren Copolymere mit Estern und Amiden von Acrylsäure und Methacrylsäure. Die unter den Bezeichnungen Merquat®100 (Poly(dimethyldiallylammoniumchlorid)) und Merquat®550 (Dimethyldiallylammoniumchlorid-Acrylamid-Copolymer) im Handel erhältlichen Produkte sind Beispiele für solche kationischen Polymere,
- Copolymere des Vinylpyrrolidons mit quaternierten Derivaten des Dialkylaminoalkylacrylats und -methacrylats, wie beispielsweise mit Diethylsulfat quaternierte Vinylpyrrolidon-Dimethylaminoethylmethacrylat-Copolymere. Solche Verbindungen sind unter den Bezeichnungen Gafquat®734 und Gafquat®755 im Handel erhältlich,
- Vinylpyrrolidon-Vinylimidazoliummethochlorid-Copolymere, wie sie unter den Bezeichnungen Luviquat® FC 370, FC 550, FC 905 und HM 552 angeboten werden,
- quaternierter Polyvinylalkohol,
   sowie die unter den Bezeichnungen
- Polyquaternium 2,
- Polyquaternium 17,
- Polyquaternium 18 und
- Polyquaternium 27 bekannten Polymeren mit quartären Stickstoffatomen in der Polymerhauptkette.

Gleichfalls als kationische Polymere eingesetzt werden können die unter den Bezeichnungen Polyquaternium-24 (Handelsprodukt z. B. Quatrisoft® LM 200), bekannten Polymere. Ebenfalls verwendbar sind die Copolymere des Vinylpyrrolidons, wie sie als Handelsprodukte Copolymer 845 (Hersteller: ISP), Gaffix® VC 713 (Hersteller: ISP), Gafquat®ASCP 1011, Gafquat®HS 110, Luviquat®8155 und Luviquat® MS 370 erhältlich sind.

Weitere kationische Polymere sind die sogenannten "temporär kationischen" Polymere. Diese Polymere enthalten üblicherweise eine Aminogruppe, die bei bestimmten pH-Werten als quartäre Ammoniumgruppe und somit kationisch vorliegt. Bevorzugt sind beispielsweise Chitosan und dessen Derivate, wie sie beispielsweise unter den Handelsbezeichnungen Hydagen® CMF, Hydagen® HCMF, Kytamer® PC und Chitolam® NB/101 im Handel frei verfügbar sind. Chitosane sind deacetylierte Chitine, die in unterschiedlichen Deacetylierungsgraden und unterschiedlichen Abbaugraden (Molekulargewichten) im Handel erhältlich sind.

Das oder die kationische(n) Polymer(e) wird (werden) in den erfindungsgemäßen Reinigungszusammensetzungen - bezogen auf deren Gesamtgewicht - bevorzugt in Mengen von 0,2 bis 3 Gew.-% und insbesondere von 0,5 bis 2 Gew.-%, eingesetzt.

Die erfindungsgemäßen Reinigungszusammensetzungen eignen sich als kosmetische Zusammensetzungen für die Reinigung der Haut und/oder der Haare, wie beispielsweise Haarshampoos, Duschgele, Duschbäder, Waschgele, Gesichtsreiniger, Handwaschmittel und/oder Schaumbäder. Als solche können sie weitere Wirk-, Hilfs- und Zusatzstoffe enthalten, die im Folgenden beschrieben werden.

Die erfindungsgemäßen Reinigungsmittel können kationische Tenside vom Typ der quartären Ammoniumverbindungen, der Esterquats und der Amidoamine enthalten. Bevorzugte quaternäre Ammoniumverbindungen sind Ammoniumhalogenide, insbesondere Chloride und Bromide, wie Alkyltrimethylammoniumchloride, Dialkyldimethylammoniumchloride und Trialkylmethylammoniumchloride, z. B. Cetyltrimethylammoniumchlorid, Stearyltrimethylammoniumchlorid, Distearyldimethylammoniumchlorid, Lauryldimethylammoniumchlorid, Lauryldimethylbenzylammoniumchlorid und Tricetylmethylammoniumchlorid, sowie die unter den INCI-Bezeichnungen Quaternium-27 und Quaternium-83 bekannten Imidazolium-Verbindungen. Die langen Alkylketten der oben genannten Tenside weisen bevorzugt 10 bis 18 Kohlenstoffatome auf.

Bei Esterquats handelt es sich um bekannte Stoffe, die sowohl mindestens eine Esterfunktion als auch mindestens eine quartäre Ammoniumgruppe als Strukturelement enthalten. Bevorzugte Esterquats sind quaternierte Estersalze von Fettsäuren mit Triethanolamin, quaternierte Estersalze von Fettsäuren mit Diethanolalkylaminen und quaternierten Estersalzen von Fettsäuren mit 1,2-Dihydroxypropyldialkylaminen. Solche Produkte werden beispielsweise unter den Warenzeichen Stepantex®, Dehyquart® und Armocare® vertrieben. Die Produkte Armocare® VGH-70, ein N,N-Bis(2-Palmitoyloxyethyl)dimethylammoniumchlorid, sowie Dehyquart® F-75, Dehyquart® C-4046, Dehyquart® L80 und Dehyquart® AU-35 sind Beispiele für solche Esterquats.

Die Alkylamidoamine werden üblicherweise durch Amidierung natürlicher oder synthetischer Fettsäuren und Fettsäureschnitte mit Dialkylaminoaminen hergestellt. Eine besonders geeignete Verbindung aus dieser Substanzgruppe stellt das unter der Bezeichnung Tegoamid® S 18 im Handel erhältliche Stearamidopropyl-dimethylamin dar.

Die kationischen Tenside werden bevorzugt in Mengen von 0,05 bis 10 Gew.-%, bezogen auf das gesamte Mittel, eingesetzt. Mengen von 0,1 bis 5 Gew.-% sind besonders bevorzugt.

In einer weiteren bevorzugten Ausführungsform kann die Wirkung der erfindungsgemäßen Zusammensetzungen durch Emulgatoren gesteigert werden. Solche Emulgatoren sind beispielsweise
- Anlagerungsprodukte von 4 bis 30 Mol Ethylenoxid und/oder 0 bis 5 Mol Propylenoxid an lineare Fettalkohole mit 8 bis 22 C-Atomen, an Fettsäuren mit 12 bis 22 C-Atomen und an Alkylphenole mit 8 bis 15 C-Atomen in der Alkylgruppe,
- Ethylenoxid- und Polyglycerin-Anlagerungsprodukte an Methylglucosid-Fettsäureester, Fettsäurealkanolamide und Fettsäureglucamide,
- C₈-C₂₂-Alkylmono- und -oligoglycoside und deren ethoxylierte Analoga, wobei Oligomerisierungsgrade von 1,1 bis 5, insbesondere 1,2 bis 2,0, und Glucose als Zuckerkomponente bevorzugt sind,
- Gemische aus Alkyl-(oligo)-glucosiden und Fettalkoholen, zum Beispiel das im Handel erhältliche Produkt Montanov®68,
- Anlagerungsprodukte von 5 bis 60 Mol Ethylenoxid an Rizinusöl und gehärtetes Rizinusöl,
- Partialester von Polyolen mit 3-6 Kohlenstoffatomen mit gesättigten Fettsäuren mit 8 bis 22 C-Atomen,
- Sterine. Als Sterine wird eine Gruppe von Steroiden verstanden, die am C-Atom 3 des Steroid-Gerüstes eine Hydroxylgruppe tragen und sowohl aus tierischem Gewebe (Zoosterine) wie auch aus pflanzlichen Fetten (Phytosterine) isoliert werden. Beispiele für Zoosterine sind das Cholesterin und das Lanosterin. Beispiele geeigneter Phytosterine sind Ergosterin, Stigmasterin und Sitosterin. Auch aus Pilzen und Hefen werden Sterine, die sogenannten Mykosterine, isoliert.
- Phospholipide. Hierunter werden vor allem die Glucose-Phospholipide, die z.B. als Lecithine bzw. Phosphatidylcholine aus z.B. Eidotter oder Pflanzensamen (z.B. Sojabohnen) gewonnen werden, verstanden.
- Fettsäureester von Zuckern und Zuckeralkoholen wie Sorbit,
- Polyglycerine und Polyglycerinderivate wie beispielsweise Polyglycerinpoly-12-hydroxystearat (Handelsprodukt Dehymuls® PGPH),
- Lineare und verzweigte Fettsäuren mit 8 bis 30 C - Atomen und deren Na-, K-, Ammonium-, Ca-, Mg- und Zn - Salze,

Die Emulgatoren werden bevorzugt in Mengen von 0,1 - 25 Gew.-%, insbesondere 0,5 - 15 Gew.-%, bezogen auf das gesamte Mittel, eingesetzt.

Prinzipiell können nichtionogene Emulgatoren mit einem HLB-Wert von 8 bis 18 eingesetzt werden. Nichtionogene Emulgatoren mit einem HLB-Wert von 10 - 15 können bevorzugt sein.

In einer weiteren Ausführungsform der Erfindung können die erfindungsgemäßen Zusammensetzungen zur weiteren Unterstützung ihrer haut- und haarpflegenden Wirkung zusätzlich Proteinhydrolysate und/oder deren Derivate enthalten.

Proteinhydrolysate sind Produktgemische, die durch sauer, basisch oder enzymatisch katalysierten Abbau von Proteinen (Eiweißen) erhalten werden.

In den erfindungsgemäßen Reinigungsmitteln können sowohl Proteinhydrolysate pflanzlichen als auch tierischen Ursprungs eingesetzt werden.

Tierische Proteinhydrolysate sind beispielsweise Elastin-, Kollagen-, Keratin-, Seiden- und Milcheiweiß-Proteinhydrolysate, die auch in Form von Salzen vorliegen können. Solche Produkte werden beispielsweise unter den Warenzeichen Dehylan® (Cognis), Promois® (Interorgana), Collapuron® (Cognis), Nutrilan® (Cognis), Gelita-Sol® (Deutsche Gelatine Fabriken Stoess & Co), Lexein® (Inolex) und Kerasol® (Croda) vertrieben.

Bevorzugt sind Proteinhydrolysate pflanzlichen Ursprungs, z. B. Soja-, Mandel-, Reis-, Erbsen-, Kartoffel- und Weizenproteinhydrolysate. Solche Produkte sind beispielsweise unter den Warenzeichen Gluadin® (Cognis), DiaMin® (Diamalt), Lexein® (Inolex) und Crotein® (Croda) erhältlich.

Wenngleich der Einsatz der Proteinhydrolysate als solche bevorzugt ist, können an deren Stelle gegebenenfalls auch anderweitig erhaltene Aminosäuregemische oder einzelne Aminosäuren wie beispielsweise Arginin, Lysin, Histidin oder Pyrroglutaminsäure eingesetzt werden. Ebenfalls möglich ist der Einsatz von Derivaten der Proteinhydrolysate, beispielsweise in Form ihrer Fettsäure-Kondensationsprodukte. Solche Produkte werden beispielsweise unter den Bezeichnungen Lamepon® (Cognis), Gluadin® (Cognis), Lexein® (Inolex), Crolastin® (Croda) oder Crotein® (Croda) vertrieben.

In den erfindungsgemäßen Reinigungsmitteln können auch kationisierte Proteinhydrolysate enthalten sein, wobei das zugrunde liegende Proteinhydrolysat vom Tier, beispielsweise aus Collagen, Milch oder Keratin, von der Pflanze, beispielsweise aus Weizen, Mais, Reis, Kartoffeln, Soja oder Mandeln, von marinen Lebensformen, beispielsweise aus Fischcollagen oder Algen, oder von biotechnologisch gewonnenen Proteinhydrolysaten, stammen kann.

Die den kationischen Derivaten zugrunde liegenden Proteinhydrolysate können aus den entsprechenden Proteinen durch eine chemische, insbesondere alkalische oder saure Hydrolyse, durch eine enzymatische Hydrolyse und/oder einer Kombination aus beiden Hydrolysearten gewonnen werden. Die Hydrolyse von Proteinen ergibt in der Regel ein Proteinhydrolysat mit einer Molekulargewichtsverteilung von etwa 100 Dalton bis hin zu mehreren tausend Dalton. Bevorzugt sind solche kationischen Proteinhydrolysate, deren zugrunde liegender Proteinanteil ein Molekulargewicht von 100 bis zu 25000 Dalton, bevorzugt 250 bis 5000 Dalton aufweist. Weiterhin sind unter kationischen Proteinhydrolysaten quaternierte Aminosäuren und deren Gemische zu verstehen. Die Quaternisierung der Proteinhydrolysate oder der Aminosäuren wird häufig mittels quarternären Ammoniumsalzen wie beispielsweise N,N-Dimethyl-N-(n-Alkyl)-N-(2-hydroxy-3-chloro-n-propyl)-ammoniumhalogeniden durchgeführt. Weiterhin können die kationischen Proteinhydrolysate auch noch weiter derivatisiert sein. Typische Beispiele für die kationischen Proteinhydrolysate und -derivate sind die unter den INCI - Bezeichnungen: Cocodimonium Hydroxypropyl Hydrolyzed Collagen, Cocodimopnium Hydroxypropyl Hydrolyzed Casein, Cocodimonium Hydroxypropyl Hydrolyzed Collagen, Cocodimonium Hydroxypropyl Hydrolyzed Hair Keratin, Cocodimonium Hydroxypropyl Hydrolyzed Keratin, Cocodimonium Hydroxypropyl Hydrolyzed Rice Protein, Cocodimonium Hydroxypropyl Hydrolyzed Silk, Cocodimonium Hydroxypropyl Hydrolyzed Soy Protein, Cocodimonium Hydroxypropyl Hydrolyzed Wheat Protein, Cocodimonium Hydroxypropyl Silk Amino Acids, Hydroxypropyl Arginine Lauryl/Myristyl Ether HCl, Hydroxypropyltrimonium Gelatin, Hydroxypropyltrimonium Hydrolyzed Casein, Hydroxypropyltrimonium Hydrolyzed Collagen, Hydroxypropyltrimonium Hydrolyzed Conchiolin Protein, Hydroxypropyltrimonium Hydrolyzed keratin, Hydroxypropyltrimonium Hydrolyzed Rice Bran Protein, Hydroxyproypltrimonium Hydrolyzed Silk, Hydroxypropyltrimonium Hydrolyzed Soy Protein, Hydroxypropyl Hydrolyzed Vegetable Protein, Hydroxypropyltrimonium Hydrolyzed Wheat Protein, Hydroxypropyltrimonium Hydrolyzed Wheat Protein/Siloxysilicate, Laurdimonium Hydroxypropyl Hydrolyzed Soy Protein, Laurdimonium Hydroxypropyl Hydrolyzed Wheat Protein, Laurdimonium Hydroxypropyl Hydrolyzed Wheat Protein/Siloxysilicate, Lauryldimonium Hydroxypropyl Hydrolyzed Casein, Lauryldimonium Hydroxypropyl Hydrolyzed Collagen, Lauryldimonium Hydroxypropyl Hydrolyzed Keratin, Lauryldimonium Hydroxypropyl Hydrolyzed Silk, Lauryldimonium Hydroxypropyl Hydrolyzed Soy Protein, Steardimonium Hydroxypropyl Hydrolyzed Casein, Steardimonium Hydroxypropyl Hydrolyzed Collagen, Steardimonium Hydroxypropyl Hydrolyzed Keratin, Steardimonium Hydroxypropyl Hydrolyzed Rice Protein, Steardimonium Hydroxypropyl Hydrolyzed Silk, Steardimonium Hydroxypropyl Hydrolyzed Soy Protein, Steardimonium Hydroxypropyl Hydrolyzed Vegetable Protein, Steardimonium Hydroxypropyl Hydrolyzed Wheat Protein, Steartrimonium Hydroxyethyl Hydrolyzed Collagen, Quaternium-76 Hydrolyzed Collagen, Quaternium-79 Hydrolyzed Collagen, Quaternium-79 Hydrolyzed Keratin, Quaternium-79 Hydrolyzed Milk Protein, Quaternium-79 Hydrolyzed Silk, Quaternium-79 Hydrolyzed Soy Protein, Quaternium-79 Hydrolyzed Wheat Protein bekannten und im Handel erhältlichen Produkte.

Ganz besonders bevorzugt sind die kationischen Proteinhydrolysate und -derivate auf pflanzlicher Basis.

Die Proteinhydrolysate und deren Derivate werden bevorzugt in Mengen von 0,01 - 10 Gew.-%, bezogen auf das gesamte Mittel, eingesetzt. Mengen von 0,1 bis 5 Gew.-%, insbesondere 0,1 bis 3 Gew.-%, sind ganz besonders bevorzugt.

Ebenfalls als vorteilhaft hat sich die Kombination der erfindungsgemäßen Zusammensetzung mit Vitaminen, Provitaminen und Vitaminvorstufen sowie deren Derivaten erwiesen.

Vitamine, Pro-Vitamine und Vitaminvorstufen, die üblicherweise den Gruppen A, B, C, E, F und H zugeordnet werden, sind bevorzugt.

Zur Gruppe der als Vitamin A bezeichneten Substanzen gehören das Retinol (Vitamin A₁) sowie das 3,4-Didehydroretinol (Vitamin A₂). Das β-Carotin ist das Provitamin des Retinols. Als Vitamin A-Komponente kommen beispielsweise Vitamin A-Säure und deren Ester, Vitamin A-Aldehyd und Vitamin A-Alkohol sowie dessen Ester wie das Palmitat und das Acetat in Betracht. Die Vitamin A-Komponente wird bevorzugt in Mengen von 0,05-1 Gew.-%, bezogen auf die gesamte Zubereitung, eingesetzt.

Zur Vitamin B-Gruppe oder zu dem Vitamin B-Komplex gehören u. a.
- Vitamin B₁ (Thiamin)
- Vitamin B₂ (Riboflavin)
- Vitamin B₃. Unter dieser Bezeichnung werden häufig die Verbindungen Nicotinsäure und Nicotinsäureamid (Niacinamid) geführt. Bevorzugt ist das Nicotinsäureamid, das bevorzugt in Mengen von 0,05 bis 1 Gew.-%, bezogen auf das gesamte Mittel, eingesetzt wird.
- Vitamin B₅ (Pantothensäure und Panthenol). Im Rahmen dieser Gruppe wird bevorzugt das Panthenol eingesetzt. Geeignete Derivate des Panthenols sind insbesondere die Ester und Ether des Panthenols sowie kationisch derivatisierte Panthenole. Einzelne Vertreter sind beispielsweise das Panthenoltriacetat, der Panthenolmonoethylether und dessen Monoacetat sowie die in der WO 92/13829 offenbarten kationischen Panthenolderivate. Die genannten Verbindungen des Vitamin B₅-Typs werden bevorzugt in Mengen von 0,05 - 10 Gew.-%, bezogen auf das gesamte Mittel, eingesetzt. Mengen von 0,1 - 5 Gew.-% sind besonders bevorzugt.
- Vitamin B₆ (Pyridoxin sowie Pyridoxamin und Pyridoxal).
- Vitamin C (Ascorbinsäure). Die übliche Einsatzmenge von Vitamin C beträgt 0,1 bis 3 Gew.-%, bezogen auf das gesamte Mittel. Die Verwendung in Form des Palmitinsäureesters, der Glucoside oder Phosphate kann bevorzugt sein. Die Verwendung in Kombination mit Tocopherolen kann ebenfalls bevorzugt sein.
- Vitamin E (Tocopherole, insbesondere α-Tocopherol). Tocopherol und seine Derivate, worunter insbesondere die Ester wie das Acetat, das Nicotinat, das Phosphat und das Succinat fallen, werden bevorzugt in Mengen von 0,05-1 Gew.-%, bezogen auf das gesamte Mittel, eingesetzt.
- Vitamin F. Unter dem Begriff "Vitamin F" werden üblicherweise essentielle Fettsäuren, insbesondere Linolsäure, Linolensäure und Arachidonsäure, verstanden.
- Vitamin H. Als Vitamin H wird die Verbindung (3aS,4S, 6aR)-2-Oxohexahydrothienol[3,4-d]-imidazol-4-valeriansäure bezeichnet, für die sich aber zwischenzeitlich der Trivialname Biotin durchgesetzt hat. Biotin wird bevorzugt in Mengen von 0,0001 bis 1,0 Gew.-%, insbesondere in Mengen von 0,001 bis 0,01 Gew.-%, eingesetzt.

Bevorzugt ist der Einsatz von Vitaminen, Provitaminen und Vitaminvorstufen aus den Gruppen A, B, E und H. Panthenol und seine Derivate sowie Nicotinsäureamid und Biotin sind besonders bevorzugt.

Weiterhin kann in einer bevorzugten Ausführungsform der Erfindung zusätzlich ein UV - Filter (I) eingesetzt werden. Die zu verwendenden UV-Filter unterliegen hinsichtlich ihrer Struktur und ihrer physikalischen Eigenschaften keinen generellen Einschränkungen. Vielmehr eignen sich alle im Kosmetikbereich einsetzbaren UV-Filter, deren Absorptionsmaximum im UVA(315-400 nm)-, im UVB(280-315nm)- oder im UVC(<280 nm)-Bereich liegt. UV-Filter mit einem Absorptionsmaximum im UVB-Bereich, insbesondere im Bereich von etwa 280 bis etwa 300 nm, sind besonders bevorzugt.

Die UV-Filter können beispielsweise ausgewählt werden aus substituierten Benzophenonen, p-Aminobenzoesäureestern, Diphenylacrylsäureestern, Zimtsäureestern, Salicylsäureestern, Benzimidazolen und o-Aminobenzoesäureestern.

Gemäß einer weiteren Ausführungsform der Erfindung sind solche UV-Filter bevorzugt, die eine kationische Gruppe, insbesondere eine quartäre Ammoniumgruppe, aufweisen.

Zwei bevorzugte UV-Filter mit kationischen Gruppen sind die als Handelsprodukte erhältlichen Verbindungen Zimtsäureamidopropyl-trimethylammoniumchlorid (Incroquat®UV-283) und Dodecyldimethylaminobenzamidopropyl-dimethylammoniumtosylat (Escalol® HP 610).

Der oder die UV-Filter (I) werden üblicherweise in Mengen 0,1-5 Gew.-%, bezogen auf das gesamte Mittel, eingesetzt. Mengen von 0,4-2,5 Gew.-% sind bevorzugt.

Neben den zwingenden Komponenten und den weiteren, zuvor genannten bevorzugten Komponenten können die erfindungsgemäßen Reinigungsmittel prinzipiell alle weiteren, dem Fachmann für solche kosmetischen Mittel bekannten Komponenten enthalten.

Weitere Wirk-, Hilfs- und Zusatzstoffe sind beispielsweise:
- Weitere Verdickungsmittel wie Gelatine oder Pflanzengumme, beispielsweise Agar-Agar, Guar-Gum, Alginate, Xanthan-Gum, Gummi arabicum, Karaya-Gummi, Johannisbrotkernmehl, Leinsamengummen, Dextrane, Stärke-Fraktionen und Derivate wie Amylose, Amylopektin und Dextrine, Tone und Schichtsilikate wie z. B. Bentonit oder vollsynthetische Hydrokolloide wie z. B. Polyvinylalkohol, die Ca-, Mg- oder Zn - Seifen,
- Strukturanten wie Maleinsäure und Milchsäure,
- Lösungsmittel und -vermittler wie Ethanol, Isopropanol, Ethylenglykol, Propylenglykol, Glycerin und Diethylenglykol,
- faserstrukturverbessernde Wirkstoffe, insbesondere Mono-, Di- und Oligosaccharide wie beispielsweise Glucose, Galactose, Fructose, Fruchtzucker und Lactose,
- Farbstoffe zum Anfärben des Mittels,
- weitere Substanzen zur Einstellung des pH-Wertes, wie beispielsweise α- und β-Hydroxycarbonsäuren,
- Wirkstoffe wie Allantoin und Bisabolol,
- Komplexbildner wie EDTA, NTA, β-Alanindiessigsäure und Phosphonsäuren,
- Ceramide. Unter Ceramiden werden N-Acylsphingosin (Fettsäureamide des Sphingosins) oder synthetische Analogen solcher Lipide (sogenannte Pseudo-Ceramide) verstanden,
- Trübungsmittel wie Latex, Styrol/PVP- und Styrol/Acrylamid-Copolymere,
- Perlglanzmittel wie Ethylenglykolmono- und -distearat sowie PEG-3-distearat,
- Pigmente,
- Treibmittel wie Propan-Butan-Gemische, N₂O Dimethylether, CO₂ und Luft,
- Viskositätsregler wie Salze (NaCl).

Ein zweiter Gegenstand der Erfindung ist die Verwendung des erfindungsgemäßen Reinigungsmittels für die Reinigung und Pflege von Haut und Haaren.

Die Reinigungsmittel der vorliegenden Erfindung schäumen in Verbindung mit Wasser schnell auf und liefern eine hohe Schaummenge. Der Schaum ist stabil und lässt sich leicht auf der Haut / den Haaren verteilen. Durch seine geringe Porengröße fühlt sich der Schaum cremig an, was den Pflegeeffekt während des Reinigungsvorgangs spürbar werden lässt. Des Weiteren lässt sich der Schaum nach der Reinigung schnell und gründlich wieder abspülen, ohne ein glitschiges Gefühl auf der Anwendungsoberfläche zu hinterlassen.

Die erfindungsgemäßen Reinigungsmittel sind weiterhin stabil und zeigen auch bei längerer Lagerung keine Separationserscheinungen.

### Beispiele

Es wurde die folgende erfindungsgemäße Reinigungszusammensetzung hergestellt:

| **Rohstoff** | **Menge** |
|---|---|
| **Fettalkoholpolyglycolethersulfat (C12-14), 2EO, 70% AS** | 10 |
| **Comperlan®¹ 100** | 0,5 |
| **Kokosamidopropylbetain 40% AS** | 10 |
| **Polyethylenglycol (PEG-32)** | 0,25 |
| **Herbasol-Extrakt Weißer Tee®²** | 0,1 |
| **Benecel®³ MP 333C** | 0,25 |
| **Natriumbenzoat** | 0,4 |
| **Cetiol®⁴ HE** | 0,5 |
| **CP Styrol-Acrylsäure OP 40%** | 1 |
| **Citronensäure** | 0,25 |
| **NaCl** | 0,4 |
| **Parfum** | 1 |
| **Wasser** | ad 100 |

Die Haut fühlte sich nach der Reinigung mit der erfindungsgemäßen Reinigungszusammensetzung weich und gepflegt an.

Die Reinigungszusammensetzung ließ sich sehr gut aufschäumen und auf der Haut verteilen.

Die Reinigungszusammensetzung wurde hinsichtlich ihrer Schaumeigenschaften wie Schaummenge, Porengröße, Cremigkeit, Festigkeit, Stabilität und Abspülbarkeit des Schaums von einer Gruppe von Experten in einem Blindtest gegenüber einer Marktrezeptur getestet, die anstelle des nichtionischen Celluloseethers b) (Benecel®³ MP 333C) und des nichtionischen Polymers c) (PEG-32) ein kationisches Polymer enthält.

Der Blindtest ergab, dass die Wirkstoffmischung in der erfindungsgemäßen Reinigungszubereitung die zusätzliche Einarbeitung klassischer Pflegekomponenten wie kationischer Polymere überflüssig macht, denn die erfindungsgemäße Rezeptur und die auf einem kationischen Polymer basierende Rezeptur wurden von den Experten in Bezug auf die zuvor genannten Eigenschaften als gleichwertig wahrgenommen.

Es wurden die folgenden Handelsprodukte eingesetzt:
- 1: INCI-Bezeichnung: Cocamide MEA; Cognis
- 2: INCI-Bezeichnung: Isopropylmyristate, Camellia Sinensis Leaf Extract; Cosmetochem,
- 3: INCI-Bezeichnung: Hydroxypropyl Methylcellulose; Hercules
- 4: INCI-Bezeichnung: PEG-7 Glyceryl Cocoate; Cognis

## Patentansprüche

1. Kosmetisches Reinigungsmittel, enthaltend
a) mindestens ein mildes anionisches und mindestens ein mildes amphoteres/zwitterionisches Tensid, wobei die Gesamtmenge an mildem(n) anionischem(n) und mildem(n) amphoterem(n)/zwitterionischem(n) Tensid(en) 3 bis 20 Gew.-% beträgt,
b) 0,005 bis 5 Gew.-% mindestens eines nichtionischen Celluloseethers und
c) 0,005 bis 5 Gew.-% mindestens eines nichtionischen Polymers der Formel (I) 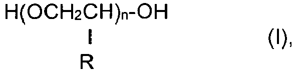 in der R für ein Wasserstoffatom oder eine Methylgruppe, und n für einen durchschnittlichen Wert von 10 bis 600 steht.

2. Reinigungsmittel nach Anspruch 1, **dadurch gekennzeichnet, dass** das Verhältnis von anionischem(n) Tensid(en) zu dem(n) amphoteren/zwitterionischen Tensid(en) 3 : 1 bis 1 : 2, bevorzugt 2,5 : 1 bis 1 : 1,5 und insbesondere 2 : 1 bis 1 : 1 beträgt.

3. Reinigungsmittel nach einem der Ansprüche 1 oder 2, **dadurch gekennzeichnet dass** das (die) anionische(n) Tensid(e) ausgewählt ist (sind) aus Alkylpolyglykolethersulfaten, Ethercarbonsäuren und/oder Sulfobernsteinsäuremono-alkylpolyoxyethylestern mit 8 bis 18 C-Atomen in der Alkylgruppe und 1 bis 10, bevorzugt mit 1 bis 4 Oxyethylgruppen und das (die) amphotere(n) /zwitterionische(n) Tensid(e) ausgewählt ist (sind) aus C₈₋₁₈-Alkylbetainen, C₈₋₁₈-Alkylamido(C₁₋₄)-alkylbetainen und/oder C₈₋₁₈-Alkylamphomono- oder-diacetaten.

4. Reinigungsmittel nach einem der Ansprüche 1 bis 3, **dadurch gekennzeichnet, dass** es - bezogen auf sein Gewicht - 0,01 bis 4 Gew.-%, bevorzugt 0,05 bis 3 Gew.-% und insbesondere 0,1 bis 2 Gew.-% eines nichtionischen Celluloseethers b) enthält, der vorzugsweise aus der Gruppe der Ether und Mischether und insbesondere aus Hydroxyethylcellulose, Hydroxypropylcellulose oder Hydroxypropyl Methylcellulose sowie aus Mischungen dieser Ether ausgewählt ist.

5. Reinigungsmittel nach Anspruch 4, **dadurch gekennzeichnet, dass** es als Komponente b) Hydroxypropyl Methylcellulose enthält.

6. Reinigungsmittel nach einem der Ansprüche 1 bis 5, **dadurch gekennzeichnet, dass** es - bezogen auf sein Gewicht - 0,01 bis 4 Gew.-%, bevorzugt 0,05 bis 3 Gew.-% und insbesondere 0,1 bis 2 Gew.-% eines nichtionischen Polymers c) der Formel (I) enthält, das bevorzugt ausgewählt ist aus der Gruppe der Polyethylenglycole, in denen n einen Wert von 10 bis 400 aufweist.

7. Reinigungsmittel nach einem der Ansprüche 1 bis 6, **dadurch gekennzeichnet, dass** es - bezogen auf sein Gewicht - zusätzlich 0,01 bis 5 Gew.-%, bevorzugt 0,05 bis 4 Gew.-%, mehr bevorzugt 0,1 bis 3 Gew.-% und insbesondere 0,2 bis 2 Gew.-% eines Alkanolamids der Formel (II) enthält, 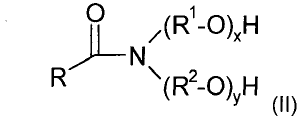 in der R eine C₈-C₂₄ gesättigte oder ungesättigte, lineare oder vernetzte aliphatische Gruppe, R₁ und R₂ gleich oder verschieden sind und eine C₂-C₄ lineare oder verzweigte aliphatische Gruppe bilden, x einen Wert von 0 bis 10 und y einen Wert von 1 bis 10 aufweist, wobei die Summe aus x+y kleiner oder gleich 10 ist.

8. Reinigungsmittel nach einem der Ansprüche 1 bis 7, **dadurch gekennzeichnet, dass** es zusätzlich mindestens einen Lösungsvermittler aus der Gruppe der Monoester und/oder Gemische aus Monoestern und Diestern des Glycerins mit verzweigten, oder geradkettigen, gesättigten oder ungesättigten Fettsäuren einer C-Kettenlänge von 8 bis 24, bevorzugt von 10 bis 18 und insbesondere von 12 bis 16 enthält, die einen Ethoxylierungsgrad von 1 bis 20, bevorzugt von 2 bis 17, besonders bevorzugt von 4 bis 13 und insbesondere von 6 bis 10 aufweisen.

9. Verwendung eines Reinigungsmittels nach einem der Ansprüche 1 bis 8 für die Reinigung und Pflege von Haut und Haaren.

## Claims

1. A cosmetic cleansing agent, containing
a) at least one mild anionic and at least one mild amphoteric/zwitterionic surfactant, wherein the total quantity of mild anionic and mild amphoteric/zwitterionic surfactant(s) is from 3 to 20 wt.%,
b) from 0.005 to 5 wt.% of at least one non-ionic cellulose ether and
c) from 0.005 to 5 wt.% of at least one non-ionic polymer of formula (I) 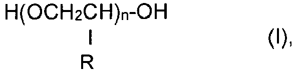 in which R denotes a hydrogen atom or a methyl group and n denotes an average value of from 10 to 600.

2. The cleansing agent according to claim 1, **characterized in that** the ratio of anionic surfactant(s) to the amphoteric/zwitterionic surfactant(s) is 3:1 to 1:2, preferably 2.5:1 to 1:1.5 and in particular 2:1 to 1:1.

3. The cleansing agent according to one of claims 1 or 2, **characterized in that** the anionic surfactant(s) is (are) selected from the group consisting of alkyl polyglycol ether sulfates, ether carboxylic acids and/or sulfosuccinic acid monoalkyl polyoxyethyl esters having from 8 to 18 carbon atoms in the alkyl group and from 1 to 10, preferably from 1 to 4, oxyethyl groups, and the amphoteric/zwitterionic surfactant(s) is (are) selected from C₈₋₁₈ alkyl betaines, C₈₋₁₈ alkyl amido(C₁₋₄)alkyl betaines and/or C₈₋₁₈ alkyl amphomono- or -diacetates.

4. The cleansing agent according to one of claims 1 to 3, **characterized in that** it contains, based on its weight, from 0.01 to 4 wt.%, preferably from 0.05 to 3 wt.% and in particular from 0.1 to 2 wt.% of a non-ionic cellulose ether b), which is preferably selected from the group of ethers and mixed ethers and in particular of hydroxyethyl cellulose, hydroxypropyl cellulose or hydroxypropyl methyl cellulose and mixtures of said ethers.

5. The cleansing agent according to claim 4, **characterized in that** it contains hydroxypropyl methyl cellulose as component b).

6. The cleansing agent according to one of claims 1 to 5, **characterized in that** it contains, based on its weight, from 0.01 to 4 wt.%, preferably from 0.05 to 3 wt.% and in particular from 0.1 to 2 wt.% of a non-ionic polymer c) of formula (I), which is preferably selected from the group of the polyethylene glycols, in which n has a value of from 10 to 400.

7. The cleansing agent according to one of claims 1 to 6, **characterized in that** it additionally contains, based on its weight, from 0.01 to 5 wt.%, preferably from 0.05 to 4 wt.%, more preferably from 0.1 to 3 wt.% and in particular from 0.2 to 2 wt.% of an alkanolamide of formula (II), 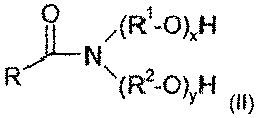 in which R is a C₈-C₂₄ saturated or unsaturated, linear or crosslinked aliphatic group, R₁ and R₂ are the same or different and form a C₂-C₄ linear or branched aliphatic group, x has a value of from 0 to 10 and y has a value of from 1 to 10, the sum of x + y being less than or equal to 10.

8. The cleansing agent according to one of claims 1 to 7, **characterized in that** it additionally contains at least one solubilizer from the group of monoesters and/or mixtures of monoesters and diesters of glycerol with branched or straight-chain, saturated or unsaturated fatty acids having a C chain length of from 8 to 24, preferably from 10 to 18 and in particular from 12 to 16, which fatty acids have a degree of ethoxylation of from 1 to 20, preferably from 2 to 17, particularly preferably from 4 to 13 and in particular from 6 to 10.

9. The use of a cleansing agent according to one of claims 1 to 8 for cleansing and nourishing skin and hair.

## Revendications

1. Agent nettoyant cosmétique contenant :
a) au moins un tensioactif anionique doux et au moins un tensioactif amphotère/zwitterionique doux, dans lequel la quantité totale de tensioactif(s) anionique(s) doux et de tensioactif(s) amphotère(s)/zwittérionique(s) doux se situe dans la plage allant de 3 à 20 % en poids,
b) de 0,005 à 5 % en poids d'au moins un éther de cellulose non-ionique et
c) de 0,005 à 5 % en poids d'au moins un polymère non-ionique de Formule (I) 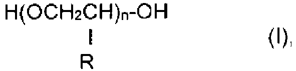 dans laquelle R représente un atome d'hydrogène ou un groupe méthyle, et n est une valeur moyenne située dans la plage allant de 10 à 600.

2. Agent nettoyant selon la revendication 1, **caractérisé en ce que** le rapport du/des tensioactif(s) anionique(s) au(x) tensioactif(s) amphotère(s)/zwittérionique(s) se situe dans la plage allant de 3:1 à 1:2, de préférence de 2,5:1 à 1:1,5 et en particulier de 2:1 à 1:1.

3. Agent nettoyant selon l'une des revendications 1 ou 2, **caractérisé en ce que** le(s) tensioactif(s) anionique(s) est/sont choisi(s) parmi les sulfates d'alkylpolyglycoléther, les acides éthercarboxyliques et/ou les alkylpolyoxyéthylesters d'acide sulfosuccinique ayant de 8 à 18 atomes de carbone dans le groupe alkyle et de 1 à 10, de préférence de 1 à 4 groupes éthoxy, et **en ce que** le(s) tensioactif(s) amphotère(s)/zwittérionique(s) est/sont choisi(s) parmi (s) les bétaïnes de C₈₋₁₈-alkyle, les bétaïnes de C₈₋₁₈-alkylamido(C₁₋₄)-alkyle et/ou les C₈₋₁₈-alkylamphomono- ou -di-acétates.

4. Agent nettoyant selon l'une quelconque des revendications 1 à 3, **caractérisé en ce qu'**il contient - par rapport à son poids - de 0,01 à 4 % en poids, de préférence de 0,05 à 3 % en poids, et en particulier de 0,1 à 2 % en poids d'un esther de cellulose non-ionique b), lequel est de préférence choisi parmi le groupe des éthers et des éthers mixtes, et en particulier parmi l'hydroxyéthylcellulose, l'hydroxypropylcellulose ou l'hydroxypropylméthylcellulose, ainsi que les mélanges de ces éthers.

5. Agent nettoyant selon la revendication 4, **caractérisé en ce qu'**il contient, en tant que constituant b), de l'hydroxypropylméthylcellulose.

6. Agent nettoyant selon l'une quelconque des revendications 1 à 5, **caractérisé en ce qu'**il contient - par rapport à son poids - de 0,01 à 4 % en poids, de préférence de 0,05 à 3 % en poids, et en particulier de 0,1 à 2 % en poids d'un polymère non-ionique c) de Formule (I), lequel est de préférence choisi parmi le groupe des polyéthylèneglycols, où n est une valeur située dans la plage allant de 10 à 400.

7. Agent nettoyant selon l'une quelconque des revendications 1 à 6, **caractérisé en ce qu'**il contient en outre - par rapport à son poids - de 0,01 à 5 % en poids, de préférence de 0,05 à 4 % en poids, de façon plus préférée de 0,1 à 3 % en poids et en particulier de 0,2 à 2 % en poids d'un alcanolamide de Formule (II), 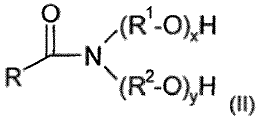 dans laquelle R est un groupe aliphatique saturé ou insaturé, linéaire ou ramifié, en C₈-C₂₄, R₁ et R₂ sont identiques ou différents et forment un groupe aliphatique linéaire ou ramifié en C₂-C₄, x a une valeur située dans la plage allant de 0 à 10 et y a une valeur située dans la plage allant de 1 à ayant 10, la somme de x + y étant inférieure ou égale à 10.

8. Agent nettoyant selon l'une quelconque des revendications 1 à 7, **caractérisé en ce qu'**il contient en outre au moins un agent solubilisant du groupe des monoesters et/ou des mélanges de monoesters et de diesters de glycérol avec des acides gras saturés ou insaturés à chaîne droite ou ramifiée, ayant une longueur de chaîne de carbone de 8 à 24, de préférence de 10 à 18 et en particulier de 12 à 16, présentant un degré d'éthoxylation de 1 à 20, de préférence de 2 à 17, de façon particulièrement préférée de 4 à 13 et en particulier de 6 à 10.

9. Utilisation d'un agent nettoyant selon l'une quelconque des revendications 1 à 8 pour le nettoyage et le soin de la peau et des cheveux.
